# EUROPEAN PATENT APPLICATION

(11) **EP 2 818 865 A1**
(43) Date of publication of application: **31.12.2014**
(21) Application number: 13174135.7
(22) Date of filing: 27.06.2013
(51) Int. Cl.: G01N 33/50

(54) **Method for the identification of effective drugs**

(71) Applicant: Alacris Theranostics GmbH, 14195 Berlin (DE)
(72) Inventor: The designation of the inventor has not yet been filed
(74) Representative: Fanelli Haag Kilger PLLC

(57) **Abstract**

The present invention relates to a method for the analysis and identification of patients responding to a specific drug, represented by tumor derived cell lines/3D tumor models, or, in case of other diseases, by appropriate patient derived cell lines (e.g. through the redifferentiation of patient derived iPS cell lines into the relevant target cell type) for the drug/disease.

The invention further relates to a method of screening for pharmaceutical effects (e.g. anti-tumor activity) and the responding patient population of a compound or mixture of compounds comprising the steps of:
(i) providing a plurality of cell lines, tissues, biopsies or samples which had been treated in a way, that each individual cell line, tissue, biopsy or sample comprises a unique genetic sequence, which acts as a label or wherein different parts of a cell line, tissue, biopsy or sample comprise a unique genetic and/or epigenetic sequence, which acts as a label and wherein the genome and/or epigenome of said cell lines, tissues, biopsies or samples is at least partially determined;
(ii) contacting said cell lines, tissues, biopsies or samples with the compound or mixture of compounds to be tested for pharmacological activity under different conditions (exposure time, concentration" ratios between different compounds;
(iii) optionally growing said cell lines, tissues, biopsies or samples in presence of the compound or mixture of compounds to be tested for pharmacological activity under different conditions;
(iv) optionally selecting a subpopulation of said cell lines, tissues, biopsies or samples responding (or non-responding) to the compound or mixture of compounds, e.g. by magnetic separation, fluorescence activated cell sorting or similar techniques;
(v) analyzing and/or sequencing the surviving, grown or selected cells and correlating the amount of identified unique labels with the activity of the pharmacological compound or mixture of compounds against the particular cell line.

## Description

### Field of the invention

The present invention is in the field of personalization of therapy through computer models and biomarker/companion diagnostic development, particularly, but not exclusively, for cancer treatment

### Background of the invention

In many cases only a fraction of patients benefit from their drug treatment. It has been estimated that typical response rates for drugs in many areas of medicine are below 50%. This is most dramatic in tumor treatment, where the average response rate to mechanistic drug treatment is estimated to be approximately 25 % (TRENDS in Molecular Medicine Vol.7 No.5 May 2001, 201) leaving most patients suffering side effects, but no or very little benefits.

This effect also contributes very significantly to the extremely high cost of the development of new drugs, not only in oncology, but also in many other areas of medicine. Drugs fail during development, since the they are not tested and later used on the particular patient population which would benefit from the drug, but on unselected (standard medicine) or only partly enriched (stratified medicine) patient populations.

In the modern world, cancer is one of the major causes of death. In order to provide continuously new therapies against cancer and other tumor diseases it is important to identify compounds with anti-tumor activity in specific patients.

It would be a great improvement, if a method was provided, which allows high throughput screening for the analysis of a new compound, a known compound or a mixture of compounds against different cell lines representing different patients, cell- or tumor types and/or diseases. Ideally such a method would allow to identify, if a subset of cell lines responds to a specific drug, and to identify methods (e.g. biomarkers or biomarker combinations/companion diagnostics and/or full computer models (ModCell)), which allow the identification of patient populations more or less likely to respond to the drug.

### Brief description of the invention

The present invention relates to a method for the analysis and identification of patients responding to a specific drug, represented by tumor derived cell lines/3D tumor models, or, in case of other diseases, by appropriate patient derived cell lines (e.g. through the redifferentiation of patient derived iPS cell lines into the relevant target cell type) for the drug/disease.

The invention further relates to a method of screening for pharmaceutical effects (e.g. anti-tumor activity) and the responding patient population of a compound or mixture of compounds comprising the steps of:
(i) providing a plurality of cell lines, tissues, biopsies or samples which had been treated in a way, that each (or essentially each) individual cell line, tissue, biopsy or sample is marked by a unique genetic sequence or where the different cell lines or samples can be distinguished by specific sequence features in their genome or epigenome.;
(ii) if the cells/cell lines only exist as pool, amplifying the pool
(iii) contacting said cell lines, tissues, biopsies or samples with the compound or mixture of compounds to be tested for pharmacological activity under different conditions (exposure time, concentrations, ratios between different compounds etc.);
(iv) optionally growing said cell lines, tissues, biopsies or samples in presence of the compound or mixture of compounds to be tested for pharmacological activity under different conditions;
(v) optionally selecting a subpopulation of said cell lines, tissues, biopsies or samples responding (or non-responding) to the compound or mixture of compounds, e.g. by magnetic separation, fluorescence activated cell sorting or similar techniques selecting for or against an appropriate phenotype reflecting the intended pharmacological effect;
(vi) analyzing and/or sequencing the surviving, grown or selected cells and correlating the amount of identified unique labels with the activity of the pharmacological compound or mixture of compounds against the particular cell line e.g. by comparison to the result with an untreated (mock treated) mixture..

### Definitions

"Cancer" herein relates to malignant neoplasia. Examples of malignant neoplasia include solid and haematological tumors. Solid tumors are exemplified by tumors of the breast, bladder, bone, brain, central and peripheral nervous system, colon, endocrine glands (e.g. thyroid and adrenal cortex), esophagus, endometrium, germ cells, head and neck, kidney, liver, lung, larynx and hypopharynx, mesothelioma, ovary, pancreas, prostate, rectum, renal, small intestine, soft tissue, testis, stomach, skin, ureter, vagina and vulva. Malignant neoplasia include inherited cancers exemplified by Retinoblastoma and Wilms tumor. In addition, malignant neoplasia include primary tumors in said organs and corresponding secondary tumors in distant organs ("tumor metastases"). Hematological tumors are exemplified by aggressive and indolent forms of leukemia and lymphoma, namely non-Hodgkins disease, chronic and acute myeloid leukemia (CML / AML), acute lymphoblastic leukemia (ALL), Hodgkins disease, multiple myeloma and T-cell lymphoma. Also included are myelodysplastic syndrome, plasma cell neoplasia, paraneoplastic syndromes, cancers of unknown primary site as well as AIDS related malignancies.

In the context of the present invention tumor relates to tumors of the breast, bladder, bone, brain, central and peripheral nervous system, colon, endocrine glands (e.g. thyroid and adrenal cortex), esophagus, endometrium, germ cells, head and neck, kidney, liver, lung, larynx and hypopharynx, mesothelioma, ovary, pancreas, prostate, rectum, renal, small intestine, soft tissue, testis, stomach, skin, ureter, vagina and vulva.

A "sample" in the meaning of the invention can be all biological tissues and all fluids such as lymph, urine, cerebral fluid, as well as populations of microorganisms coexisting with or infecting the human body. Tissues may be, e.g. epithelium tissue, connective tissue such as bone or blood, muscle tissue such as visceral or smooth muscle and skeletal muscle and, nervous tissue. The sample is collected from the patient or appropriate experimental animals and subjected to the diagnosis according to the invention.

In the context of the invention a sample, cell line, cells, biopsy, tissue or tumor is not limited to human derived examples, but any sample, cell, cell line, biopsy, tissue which can be obtained from any animal. Preferably the sample, cell line, cells, biopsy, tissue or tumor is of mammalian origin, most preferably of human origin.

Where appropriate, as for instance in the case of solid samples, the sample may need to be solubilized, homogenized, or extracted with a solvent prior to use in the present invention in order to obtain a liquid sample. A liquid sample hereby may be a solution or suspension.

In the context of the present invention, a specific class of nucleic acid may be, inter alia, RNA, DNA, cDNA (complementary DNA), LNA (locked nucleic acid), mRNA (messenger RNA), mtRNA (mitochondrial), rRNA (ribosomal RNA), tRNA (transfer RNA), nRNA (nuclear RNA), siRNA (short interfering RNA), snRNA (small nuclear RNA), snoRNA (small nucleolar RNA), scaRNA (Small Cajal Body specific RNA), microRNA, dsRNA (double-stranded RNA), ribozyme, riboswitch, viral RNA, dsDNA (double-stranded DNA), ssDNA (single-stranded DNA), plasmid DNA, cosmid DNA, chromosomal DNA, viral DNA, mtDNA (mitochondrial DNA), nDNA (nuclear DNA), snDNA (small nuclear DNA) or the like or any other class or sub-class of nucleic acid which is distinguishable from the bulk nucleic acid in a sample.

In the context of the present invention the term "transfection" relates to any method of the deliberate incorporation of nucleic acids into eukaryotic cells. In the context of the invention the term transfection comprises chemical, non-chemical, particle based and viral methods for the introduction of nucleic acids. It might be used synonymously with the terms transformation and transduction in the context of the present invention...

Nucleic-acid amplification can be accomplished by any of the various nucleic-acid amplification methods known in the art, including but not limited to the polymerase chain reaction (PCR), ligase chain reaction (LCR), transcription-based amplification system (TAS), nucleic acid sequence based amplification (NASBA), rolling circle amplification (RCA), transcription-mediated amplification (TMA), self-sustaining sequence replication (3SR), multiple displacement amplification (MDA), recombinase polymerase amplification (RPA), helicase-dependent amplification (HDA), loop mediated isothermal amplification (LAMP), and Qβ amplification.

In the context of the present invention the term whole genome amplification (WGA) relates to the largely unbiased amplification of the entire genome of a given cell. Methods for WGA include but are not limited to isothermal amplification techniques such as MDA or cyclic amplification techniques such as Degenerate Oligonucleotide PCR (DOP-PCR), Primer Extension Preamplification (PEP), Multiple Annealing and Looping Based Amplification Cycles (MALBAC), or Nextera technology based on a transposition reaction called 'tagmentation' followed by PCR. The amplification bias can be further reduced by performing the reaction in compartments such as water in oil emulsions.

DNA sequencing techniques are of major importance in a wide variety of fields ranging from basic research to clinical diagnosis. The results available from such technologies can include information of varying degrees of specificity. For example, useful information can consist of determining whether a particular polynucleotide differs in sequence from a reference polynucleotide, confirming the presence of a particular polynucleotide sequence in a sample, determining partial sequence information such as the identity of one or more nucleotides within a polynucleotide, determining the identity and order of nucleotides within a polynucleotide, etc. The sequencing step is preferably done by means of next generation sequencing. Manufacturers and technologies are Solexa/Illumina which generate up to 600 Gigabase (Gb) of 36 or 150 bp, Roche/454 which generate up to 700 Mbp reads of 400-1000 bp, ABI/SOLiDTM which generate > 20 Gb/day reads of 35-75 bp, Helicos which generate 21 - 35 Gb reads of 25-45 bp and Complete Genomics (a service company). Other manufacturers include Pacific Bioscience commercializing PacBio RS.

The Solexa/Illumina sequencing by synthesis technology is based on reversible dye-terminators. DNA molecules are first attached to primers on a slide and amplified so that local clonal colonies are formed (bridge amplification). Four types of reversible terminator bases (RT-bases) are added, and non-incorporated nucleotides are washed away. Unlike pyrosequencing, the DNA can only be extended one nucleotide at a time. A camera takes images of the fluorescently labelled nucleotides, then the dye along with the terminal 3' blocker is chemically removed from the DNA, allowing the next cycle (Brenner et al., Nature Biotechnol. 2000. 18(6):630-634).

The SOLiD^{™} ("Sequencing by Oligonucleotide Ligation and Detection") method (Life Technologies; WO 06/084132 A2) is based on the attachment of PCR amplified fragments of template nucleic acids via universal adapter sequences to magnetic beads and subsequent detection of the fragment sequences via ligation of labelled probes to primers hybridized to the adapter sequences. For the read-out a set of four fluorescently labelled di-base probes are used. After read-out, parts of the probes are cleaved and new cycles of ligation, detection and cleavage are performed. Due to the use of di-base probes, two rounds of sequencing have to be performed for each template sequence. Other methods and devices include Ion Torrent and Roche's 454.

PacBio RS is a single molecule real-time sequencing (SMRT) platform based on the properties of zero-mode waveguides (ZMW). A single DNA polymerase enzyme is affixed at the bottom of a ZMW with a single molecule of DNA as a template. The ZMW is a structure that creates an illuminated observation volume that is small enough to observe only a single nucleotide of DNA being incorporated by DNA polymerase. Each of the four DNA nucleotides is attached to one of four different fluorescent dyes. When a nucleotide is incorporated by the DNA polymerase, the fluorescent tag is cleaved off and diffuses out of the observation area of the ZMW where its fluorescence is no longer observable. A detector detects the fluorescent signal of the nucleotide incorporation, and the base call is made according to the corresponding fluorescence of the dye.

GridION and MinION systems developed by Oxford Nanopore Technologies use biological nanopores to sequence single molecules in a so-called DNA strand sequencing approach. A mutant variant of the pore protein, alpha-hemolysin, is inserted in a synthetic proprietary polymer bilayer that restricts ion passage to the pores alone. ONT uses a special enzyme to process the DNA, not the DNA polymerases currently used by some academic teams. The enzyme is capable of ratcheting DNA through the pore at upwards of 1,000 bases/second, a rate that is controlled by various cofactors in solution. The sequence is read by monitoring an electrical signal as a strand of DNA passes through the pore protein. An informatics system reads overlapping nucleotide triplets in the middle of the pore to deduce the sequence. By engineering a hairpin at the end of the molecule, both strands of the template DNA can be sequenced in succession on the same pore. This redundancy is used to reduce the error rate to an acclaimed 4%. Also these sequencing methods are claimed in the method herein.

### Detailed description of the invention

The present invention relates to a method for the analysis and identification of patients responding to a specific drug, represented by tumor derived cell lines/3D tumor models, or, in case of other diseases, by appropriate patient derived cell lines (e.g. through the redifferentiation of patient derived iPS cell lines into the relevant target cell type) or populations of microorganisms coexisting with or infecting human beings for the drug/disease.

The invention further relates to a method of screening for pharmaceutical effects (e.g. anti-tumor activity) of a compound or mixture of compounds and the responding patient population, which is responding to the drug or not responding to the drug or showing specific side effects comprising the steps of:
(i) providing a plurality of cell lines, tissues, biopsies or samples which had been treated in a way, that each individual cell line, tissue, biopsy or sample is labeled by a unique genetic
   sequence or sequence combination or the modification pattern of preferentially a unique genetic sequence or sequence combination, either by separate cell lines, each of which carries a unique label, or a pool of such cells, into which unique labels have either been introduced, or are already present naturally
(ii) optionally, if starting with a pool of unique cells, amplifying this pool
(iii) contacting said cell lines, tissues, biopsies or samples with the compound or mixture of compounds to be tested for pharmacological activity under different conditions (exposure time, concentration-ratios between different compounds);
(iv) optionally growing said cell lines, tissues, biopsies or samples in presence of the compound or mixture of compounds to be tested for pharmacological activity under different conditions;
(v) optionally selecting a subpopulation of said cell lines, tissues, biopsies or samples responding (or non-responding) to the compound or mixture of compounds, e.g. by magnetic separation, fluorescence activated cell sorting or similar techniques;
(vi) analyzing and/or sequencing the surviving, grown or selected cells and correlating the amount of identified unique labels with the activity of the pharmacological compound or mixture of compounds against the particular cell line
vi) optionally identifying the cell lines or cells carrying the tag sequences or sequence modifications indicating relevant behavior (response, non-response, side effects etc) for further analysis optionally isolating said cell lines or cells, tissues, samples or biopsies.

Within this description the term cell line also comprises tissue, biopsies and samples as well.

Preferably the plurality of cell lines, tissues, biopsies or other samples is derived from a plurality of patients. Preferably the patients are mammals; most preferably the patients are humans.

In a preferred embodiment of the invention each cell line, tissue, biopsy or sample has a known genotype. In a more preferred embodiment of the invention each cell line is completely sequenced. In another preferred embodiment of the invention the genotype of each cell line is known and is derived from a different patient and/or comprises a different genotype and/or is infected with a different disease and/or is a different type of tumor cell line or can be determined, after the relevant cell type has been identified through the tag sequence.

In another preferred embodiment of the invention the epigenome e.g. methylation pattern of each cell line is known and is derived from a different patient and/or comprises a different epigenome.

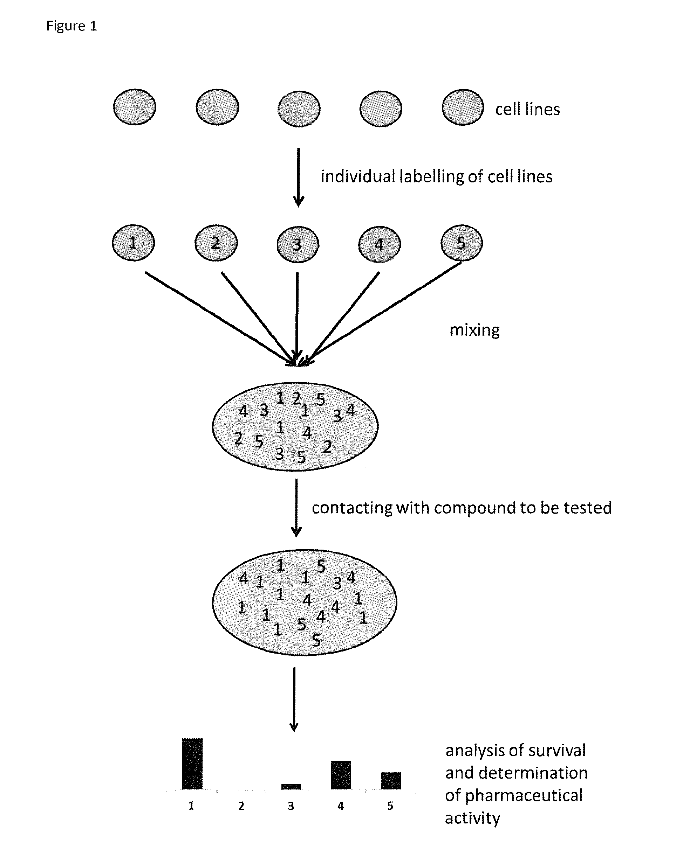

The general principle of the invention is depicted in figures 1 and 2. In general the invention relates on the analysis of the plurality of different labels, which result from different reactions of the different cell lines, tissues, biopsies or samples to the compound or mixture of compounds. Labels of cell lines which are sensitive against the compound will be underrepresented, while labels of cell lines, which are resistant against the compound or mixture of compounds will be overrepresented.

In a preferred embodiment the invention relates to the use of a plurality of cell lines tissues, biopsies or samples. The cell lines, tissues, biopsies or samples may be derived from any kind of cells, e.g. tumor cells, either malignant or benign. The plurality of cell lines might be derived from any kind of cell, e.g. liver cells or spleen cells. In the case of tumors, preferably the cell lines or samples are derived from a 3D-cell culture.

For use in the present invention each of the cell lines, tissues, biopsies or samples have to be labeled with a unique nucleic acid label or have to naturally contain such a sequence. The label might be a short known nucleic acid sequence. Preferably the label is transfected into the cell line. More preferably, the label is integrated into the cell lines via stable transfection.

The person skilled in the art readily realizes several methods for the treatment of the cell lines, tissues, biopsies or samples to introduce labels. Preferably the method for the introduction of the label is a method of transfection or transduction. More preferably the method for introduction is viral transfection. In the most preferred embodiment the label is introduced by transduction with a Lentivirus.

The nucleic acid sequences used as a label should be long enough to allow sure identification of the corresponding cell line. Preferably the nucleic acid sequences additionally comprise an additional nucleic acid sequences, which allows selective amplification, and can act as a binding site for a sequencing primer. More preferably the sequence, which can act as a binding site for a sequencing primer is identical in all labels.

In another embodiment of the invention, the cell lines are additionally labeled with fluorescence markers, which would allow additional marking of the cells. The fluorescence marker may be used to assess the efficiency of sequence label transfection and/or stable genomic integration. In yet another embodiment of the invention the fluorescently labeled cells are sorted to provide a defined titer of corresponding transfected cell lines.

Within the meaning of this invention the term compound is not limited to chemical compounds, but also includes other "compounds" to be tested for pharmaceutical activity such as biomolecules, radiation, organisms, viruses, nanoparticles, nanostructured surfaces or means of energy transfer. For example the present invention could be used to rapidly analyze the influence of X-rays or viruses on different cell lines.

The invention is neither limited to either new compounds nor to already known compounds. The invention allows the simultaneous test of a known compound, with known pharmaceutical activities suitable to treat at least one cell line, preferably with a known genotype. Alternatively the invention allows the test of a new compound for pharmaceutical activity against a variety of cell lines. If a mixture of compounds is to be tested in one embodiment of the invention the mixture comprises at least one compound with a known pharmaceutical activity.

In another embodiment of the invention the compound or mixture of compounds comprises compounds without known pharmaceutical activity or is untested for pharmaceutical activity.

The mixing of the cell lines, tissues, biopsies or samples may be done by any possible way.

In a preferred embodiment the cell lines, tissues, biopsies or samples are grown in a suitable growth medium prior to contact with the compound to be tested. In a more preferred embodiment of the invention the cell lines, tissues, biopsies or samples are grown to a defined cell count or optical density prior to contact with the compound to be tested.

Contacting the cell lines with the compound or mixture of compounds to be tested in the context of the present invention means that the compound is able to interact with the plurality of cell lines. Potential interactions include but are not limited to: dissolving the compound in the growth medium, continuous or sequential exposure to the compound (e.g. radiation), injecting the compound directly into the mixture or direct contact of the cell lines with the compound.

In one embodiment of the invention the method additionally includes the growth of the cell lines in presence of the compound or mixture of compounds to be tested. In a more preferred embodiment of the invention the method includes the growth of the cell lines for a defined period of time or to a defined cell count or optical density.

In another preferred embodiment the method includes a control of the same composition of cell lines that will not be contacted with the compound or mixture of compounds, but is otherwise treated in the same way, and can therefore act as a control.

If the growth rates of the different cell lines are known, no control is necessary, hence in one embodiment the method is performed without a set of reference cell lines, which had not been contacted with the compound.

In another preferred embodiment the method includes a set of differently labeled cell lines, which were are not diseased or are derived from a tumor, to further act as a reference for the specificity of the compound or mixture of compounds.

In a preferred embodiment of the invention contacting the cell lines with the compound or mixture of compounds comprises: growing the tumor cell line in a suitable growth medium, in which the compound to be tested is dissolved, suspended, activated, or released for a suitable time, preferably 1, 2, 3, 4, 5, 6, 7, 8, 9, 10 hours, days, weeks or months.

The present invention further allows varying the concentrations of the compound or mixtures of compounds and therefore allows the parallel testing of different concentrations of a compound or compound mixtures against a plurality of different cell lines.

After the tumor cell lines have been contacted and optionally grown in presence of the compound or mixture of compounds to be tested the activity is analyzed by the detection of the individual nucleic acid labels present in the mixture.

In a preferred embodiment of the invention dead cells and released DNA are removed, e.g. by the sorting and/or usage of enzymes with nuclease activity.

The present invention is not limited to a simple life/death screening. In one embodiment of the invention a subpopulation of the cell lines is selected and separated. In one preferred embodiment a subpopulation comprising cells responding to the compound is selected. In another preferred embodiment a subpopulation comprising cells non-responding to the compound is selected, but any other phenotype allowing selection or differential growth of cells of the appropriate phenotype increase or decrease by the drug can be used.

Methods for the selection include but are not limited to magnetic separation, fluorescence activated cell sorting or similar techniques.

The person skilled in the art readily knows several ways to identify and detect the labels. These methods include but are not limited to: Northern- respective Southern Blot, hybridization with fluorescently labeled probes, microarray analysis, amplification of the labels and targeted sequencing or whole genome sequencing.

In a preferred embodiment of the invention the detection is performed by amplifying the individual label sequences, by hybridization to a preferably fluorescent labeled probe, by detecting the label sequences by sequencing in whole genome amplifications or combinations thereof.

According to the present invention, the amount of detected label correlates anti-proportionally to the effectiveness of the tested compound against a particular cell line, especially in comparison to the untreated control. Since the genotypes or preferably whole genomes were known, the presence or absence of a particular label possible as well can be correlated with the activity of the compound against particular genotypes.

Alternatively if the cell lines were selected prior to analysis the detected labels directly correspond to the pharmaceutical effectiveness of the compound or mixture of compounds.

### Brief description of the figures

**Figure 1****:** General principle of the present invention. A plurality of cell lines is provided and each cell line is individually labeled. After the labeling the cell lines are mixed and contacted with a compound or mixture of compounds, which is to be tested for pharmaceutical activity. After cell growth in presence of the compound or mixture of compounds the survival of the different cell lines is analyzed and correlated with the effectiveness of the compound or mixture neu against the particular cell line.

## Claims

1. A method for the analysis and identification of cell lines responding to a specific compound or mixture of compounds comprising the steps of:
(i) providing a plurality of cell lines, tissues, biopsies or samples which had been treated in a way, that each individual cell line, tissue, biopsy or sample is labeled by a unique genetic sequence or sequence combination or the modification pattern of preferentially a unique genetic sequence or sequence combination, either by separate cell lines, each of which carries a unique label, or a pool of such cells, into which unique labels have either been introduced, or are already present naturally
(ii) optionally, if starting with a pool of unique cells, amplifying this pool
(iii) contacting said cell lines, tissues, biopsies or samples with the compound or mixture of compounds to be tested for pharmacological activity under different conditions (exposure time, concentration-ratios between different compounds);
(iv) optionally growing said cell lines, tissues, biopsies or samples in presence of the compound or mixture of compounds to be tested for pharmacological activity under different conditions;
(v) optionally selecting a subpopulation of said cell lines, tissues, biopsies or samples responding (or non-responding) to the compound or mixture of compounds, e.g. by magnetic separation, fluorescence activated cell sorting or similar techniques;
(vi) analyzing and/or sequencing the surviving, grown or selected cells and correlating the amount of identified unique labels with the activity of the pharmacological compound or mixture of compounds against the particular cell line;
(vi) optionally identifying the cell lines or cells carrying the tag sequences or sequence modifications indicating relevant behavior (response, non-response, side effects etc) for further analysis optionally isolating said cell lines or cells, tissues, samples or biopsies.

2. The method according to claim 1, wherein the plurality of cell lines is derived from different patients and/or from different cell types.

3. The method according to claim 1 or 2, wherein the treatment of the cell lines involves stable transfection with a nucleic acid comprising a unique DNA sequence, which acts as a label.

4. The method according to claim 3, wherein the nucleic acid is DNA or RNA.

5. The method according to any of the claims 1 to 4, wherein the plurality of cell lines, tissues, biopsies or samples are derived from 3D cell cultures.

6. The method according to any of the claims 1 to 5, wherein the compound or mixture to be tested is or comprises at least one compound with known pharmaceutical activity.

7. The method according to any of the claims 1 to 5, wherein the compound or mixture of compounds to be tested has no known pharmaceutical activity or is untested for pharmaceutical activity.

8. The method according to any of the claims 1 to 7, wherein the cell lines are contacted with the compound or mixture of compounds to be tested, by growing them in an appropriate growth medium comprising the compound or mixture thereof to be tested.

9. The method according to any of the claims 1 to 8, wherein the sequencing in the last step is whole genome sequencing.

10. The method according to any of the claims 1 to 8, wherein the sequencing in the last step is transcriptome sequencing.

11. The method according to any of the claims 1 to 10 wherein the correlation of the pharmaceutical activity to the labels additionally includes a correlation to the genotype of the cell lines, against which the compound shows activity to identify a potential mechanism of effect or target of the compound or mixture of compounds.
